# FASCICULE DE BREVET EUROPEEN

(11) **EP 2 691 071 B1**
(45) Date de publication et mention de la délivrance du brevet: **01.08.2018**
(21) Numéro de dépôt: 12706632.2
(22) Date de dépôt: 26.01.2012
(51) Int. Cl.: A61K 8/81, A61K 8/91, A61Q 5/02

(54) **POLYMERES PEIGNES POUR LES CHEVEUX**
KAMMPOLYMERE FÜR DAS HAAR
COMB POLYMERS FOR HAIR

(30) Priorité: 28.03.2011 FR 1152530
(43) Date de publication de la demande: 05.02.2014
(73) Titulaire: COATEX, 69730 Genay (FR)
(72) Inventeur: SUAU, Jean-Marc, F-69480 Lucenay (FR); GUERRET, Olivier, F-46170 Pern (FR)
(74) Mandataire: Balmefrezol, Ludovic Francis Pierre
(86) Numéro de dépôt international: PCT/FR2012/050170
(87) Numéro de publication internationale: WO 2012/131201

(56) Documents cités:
- FR-A1- 2 861 399
- FR-A1- 2 873 122
- FR-A1- 2 926 558
- DATABASE WPI Week 200382 Thomson Scientific, London, GB; AN 2003-881034 XP002664385, & JP 2003 055164 A (OSAKA YUKI KAGAKU KOGYO KK) 26 février 2003 (2003-02-26)
- DATABASE WPI Week 199601 Thomson Scientific, London, GB; AN 1996-006869 XP002664386, & JP 7 285831 A (MITSUBISHI CHEM CORP) 31 octobre 1995 (1995-10-31)

## Description

La présente invention concerne le secteur des additifs mis en oeuvre dans des formulations cosmétiques destinées à être appliquées sur le cuir chevelu. Elle consiste en l'utilisation de copolymères peignes (méth)acryliques ayant des chaînes latérales du type hydroxy ou alcoxy polyalkylène glycol, dans lesquels sont présents à la fois des chaînons d'oxyde d'éthylène et d'oxyde de propylène. Ces copolymères confèrent aux shampoings dans lesquels ils sont incorporés un effet coiffant et une élimination au rinçage plus marqués que pour les copolymères peignes de l'art antérieur, fabriqués à partir d'acide (méth)acrylique et de groupements latéraux du type hydroxy ou méthoxy polyéthylène glycol (MPEG).

Dans le domaine des soins capillaires, on utilise communément des compositions à base de tensioactifs anioniques, non-ioniques et amphotères. Celles-ci sont appliquées sur cheveux mouillés et la mousse générée par massage ou friction avec les mains permet, après rinçage à l'eau, l'élimination des salissures initialement présentes.

Si ces formulations possèdent un bon pouvoir lavant, leurs propriétés cosmétiques intrinsèques restent toutefois limitées, notamment en raison du caractère relativement agressif du traitement qu'elles font subir aux cheveux : celui-ci peut entraîner des dommages plus ou moins marqués sur la fibre capillaire, notamment à travers l'élimination progressive des lipides et des protéines contenues dans le cheveu.

Aussi est-il nécessaire de mettre en oeuvre des additifs dont la vertu consiste à améliorer les propriétés cosmétiques des formulations capillaires dans lesquelles ils sont incorporés. Parmi ces propriétés, « l'effet coiffant » est particulièrement recherché : il se manifeste à travers une facilité de coiffage et de maintien des cheveux après rinçage, de même que par un apport de volume et de légèreté au niveau de la chevelure sèche.

Pour ce faire, on a depuis longtemps utilisé des polymères cationiques qui, en raison de leur forte affinité pour la fibre capillaire de nature anionique, vont avoir des effets rémanents sur la chevelure. Néanmoins, ce phénomène peut engendrer des dépôts de produit trop importants : le toucher de la chevelure devient chargé, désagréable ; les cheveux se raidissent et on observe une adhésion interfibre qui affecte le coiffage.

Une nouvelle génération de produits a été récemment mise au point, pour pallier à de tels inconvénients. Elle s'appuie sur des copolymères (méth)acryliques de structure peigne. L'utilisation de ces produits pour le soin des cheveux a été décrite dans les documents EP 1 632508 A1 et EP 2 168 991 A1. On constate que leur mise en oeuvre dans une formulation de shampoing permet non seulement d'améliorer l'effet coiffant de celui-ci, mais facilite également son élimination après rinçage à l'eau.

A travers l'expression « copolymère (méth)acrylique peigne », on entend désigner un copolymère constitué d'un squelette essentiellement linéaire et de nature (méth)acrylique, sur lequel sont greffés au moins deux segments latéraux constitués d'au moins un « macromonomère ». Le terme « macromonomère » désigne un polymère ou un copolymère hydrosoluble, et possédant au moins un groupe terminal disposant d'une fonction éthylénique insaturée.

Dans le cas des produits mis au point pour les soins capillaires et décrits dans les documents EP 1 632 508 A1 et EP 2 168 991 A1, ce macromonomère est du type méthacrylate d'hydroxy ou de méthoxy polyéthylène glycol (méthacrylate de MPEG ou MAMPEG). En résumé, les copolymères peignes décrits dans ces deux documents sont le résultat de la synthèse entre un premier monomère anionique qui peut être l'acide (méth)acrylique, et un macromonomère alcoxy ou hydroxy polyakylène glycol de formule R - (OE)ₙ - R', avec :
- OE désignant l'oxyde d'éthylène et n un entier compris entre 3 et 300,
- R désignant une fonction insaturée polymérisable comme la fonction méthacrylate,
- R' représente l'hydrogène ou un radical carboné ayant de 1 à 30 atomes de carbone.

On notera dans ces 2 documents toute l'importance apportée aux macromonomères particuliers MPEG550 et MPEG2000, respectivement méthacrylate de polyéthylène glycol de masse molaire moyenne en poids égale à 550 g/mol et 2 000 g/mol.

Or, de manière tout à fait surprenante, la Demanderesse a mis au point l'utilisation de copolymères (méth)acryliques peignes de structure différente, qui s'avèrent plus performants au niveau de l'effet coiffant sur le cheveu, et en terme d'élimination par rinçage à l'eau. Ces polymères sont constitués :
a) de 5% à 30% d'au moins un monomère qui est l'acide (méth)acrylique
b) et de 70% à 95% d'au moins un macromonomère de formule (I) :

   R-(OP)ₘ-(OE)ₙ-R' (I)

   - m et n sont des entiers non nuls inférieurs à 150, n et m étant compris entre 10 et 90,
   - OP et OE désignent respectivement l'oxyde de propylène et l'oxyde d'éthylène,
   - R désigne une fonction insaturée polymérisable,
   - R' représente l'hydrogène ou un groupement alkyle ayant de 1 à 4 atomes de carbone.

Ces structures diffèrent de celles évoquées dans les documents EP 1 632 508 A1 et EP 2 168 991 A1, en ce sens que leur macromonomère contient à la fois des chaînons oxyde d'éthylène et oxyde de propylène. Rien ne laissait supposer qu'une telle modification allait impacter de manière aussi bénéfique les propriétés d'effet coiffant et de rinçabilité des shampoings dans lesquels on incorporerait ces polymères.

En outre, on précise que le macromonomère de formule (I) est bien connu et décrit dans le brevet US 6 034 208. De plus, la fabrication des copolymères comme indiqués ci-dessus, qui sont fabriqués à partir de ce macromonomère, est bien connue de l'homme du métier, notamment dans un procédé en continu, en semi-continu ou en batch (voir les documents US 6 815 513, US 6 214 958, US 6 664 360 et US 7 232 875). Enfin, on connaissait déjà d'autres utilisations de ces structures : notamment comme additifs dans des formulations de plâtre (EP 1 377 533 A1, EP 1 615 860 A1), de ciment (FR 2 939 128 A1 et FR 2 939 428 A1) ou dans des sauces de couchage papetières (Demande française non encore publiée et déposée sous le numéro FR 10 54575).

Aussi, un premier objet de la présente invention consiste en l'utilisation de ces composés comme agent coiffant, dans une formulation cosmétique. De manière préférée, cette utilisation est caractérisée en ce que R désigne la fonction méthacrylate ou méthacryluréthane.

De manière préférée, cette utilisation est caractérisée en ce que R' désigne l'hydrogène. Les structures -(OP)ₘ-(OE)ₙ- désignent aussi bien des structures blocs que statistiques et ce, dans toute la présente Demande.

Cette utilisation est aussi caractérisée en ce que le copolymère (méth)acrylique peigne présente une masse molaire moyenne en poids comprise entre 20 000 g/mol et 6 000 000 g/mol, préférentiellement entre 40 000 g/mol et 1 000 000 g/mol.

Le copolymère (méth)acrylique peigne peut être obtenu par polymérisation radicalaire en solution, en émulsion directe ou inverse, en suspension ou précipitation dans des solvants, en présence de systèmes catalytiques et d'agents de transfert, ou encore en une polymérisation radicalaire contrôlée et préférentiellement en la polymérisation contrôlée par des nitroxydes (NMP) ou par des cobaloxymes, en la polymérisation par transfert d'atome radicalaire (ATRP), en la polymérisation radicalaire contrôlée par des dérivés soufrés, choisis parmi des carbamates, des dithioesters ou des trithiocarbonates (RAFT) ou des xanthates.

Il peut être totalement ou partiellement neutralisé par un ou plusieurs agents de neutralisation disposant d'un cation monovalent ou polyvalent, lesdits agents étant choisis préférentiellement parmi l'ammoniaque ou parmi les hydroxydes et / ou oxydes de calcium, de magnésium, ou parmi les hydroxydes de sodium, de potassium, de lithium, ou parmi les amines primaires, secondaires ou tertiaires aliphatiques et / ou cycliques telles que préférentiellement la stéarylamine, les éthanolamines (mono-, di-, triéthanolamine), la mono et diéthylamine, la cyclohexylamine, la méthylcyclohexylamine, l'amino méthyl propanol, la morpholine, et de manière préférentielle en ce que l'agent de neutralisation est choisi parmi la triéthanolamine et l'hydroxyde de sodium.

Il peut aussi être séparé en plusieurs phases, selon des procédés statiques ou dynamiques, par un ou plusieurs solvants polaires appartenant préférentiellement au groupe constitué par l'eau, le méthanol, l'éthanol, le propanol, l'isopropanol, les butanols, l'acétone, le tétrahydrofurane ou leurs mélanges.

Cette utilisation est aussi caractérisée en ce que la formulation cosmétique comprend de 0,01 % à 50 %, préférentiellement de 0,1 % à 30 % très préférentiellement de 3 % à 20 % en poids sec dudit copolymère par rapport à son poids total.

Cette utilisation est aussi caractérisée en ce que la formulation comprend au moins un constituant choisi parmi l'eau, les solvants organiques hydrophiles et préférentiellement les alcools, préférentiellement les monoalcools, linéaires ou ramifiés en C1-C6 et les polyols et les éthers de glycols préférentiellement en C2 et des aldéhydes en C2-C4 hydrophiles, les cires, les corps gras pâteux, les gommes et leurs mélanges, d'origine animale, végétale, minérale ou synthétique, les solvants organiques lipophiles, les huiles d'origine animale, végétale, minérale ou synthétique, les esters et les éthers de synthèse, les esters du pentaérythritol, les alcools gras ayant de 12 à 26 atomes de carbone, les huiles fluorées partiellement hydrocarbonées et/ou siliconées, les huiles siliconées volatiles ou non, linéaires ou cycliques, liquides ou pâteux à température ambiante, les pigments, les nacres, les charges, les colorants hydrosolubles, les colorants liposolubles, les agents de filmification, les tensioactifs, les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents propulseurs, les céramides et leurs mélanges.

Cette utilisation est aussi caractérisée en ce que la formulation se présente sous la forme d'une composition capillaire, préférentiellement pour le maintien de la coiffure ou la mise en forme des cheveux, très préférentiellement sous forme de shampooings, de gels, de lotions de mise en plis, de lotions pour le brushing, de compositions de fixation et de coiffage choisies parmi les laques ou spray, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Un autre objet de la présente invention consiste en une formulation cosmétique contenant au moins un copolymère (méth)acrylique peigne caractérisé en ce qu'il est constitué :
a) de 5% à 30% d'au moins un monomère qui est l'acide (méth)acrylique
b) et de 70% à 95% d'au moins un macromonomère de formule (I):

   R-(OP)ₘ-(OE)ₙ-R' (I)

   - m et n sont des entiers non nuls inférieurs à 150, n et m étant compris entre 10 et 90,
   - OP et OE désignent respectivement l'oxyde de propylène et l'oxyde d'éthylène,
   - R désigne une fonction insaturée polymérisable,
   - R' représente l'hydrogène ou un groupement alkyle ayant de 1 à 4 atomes de carbone.

De manière préférée, cette formulation est caractérisée en ce que, pour ledit copolymère, R désigne la fonction méthacrylate ou méthacryluréthane.

De manière préférée, cette formulation est caractérisée en ce que, pour ledit copolymère, R' désigne l'hydrogène.

Cette formulation est aussi caractérisée en ce que, pour ledit copolymère, il présente une masse molaire moyenne en poids comprise entre 20 000 g/mol et 6 000 000 g/mol, préférentiellement entre 40 000 g/mol et 1 000 000 g/mol.

Cette formulation est aussi caractérisée en ce que la formulation cosmétique comprend de 0,01 % à 50 %, préférentiellement de 0,1 % à 30 % très préférentiellement de 3 % à 20 % en poids sec dudit copolymère par rapport à son poids total.

Cette formulation est aussi caractérisée en ce qu'elle comprend au moins un constituant choisi parmi l'eau, les solvants organiques hydrophiles et préférentiellement les alcools, préférentiellement les monoalcools, linéaires ou ramifiés en C1-C6 et les polyols et les éthers de glycols préférentiellement en C2 et des aldéhydes en C2-C4 hydrophiles, les cires, les corps gras pâteux, les gommes et leurs mélanges, d'origine animale, végétale, minérale ou synthétique, les solvants organiques lipophiles, les huiles d'origine animale, végétale, minérale ou synthétique, les esters et les éthers de synthèse, les esters du pentaérythritol, les alcools gras ayant de 12 à 26 atomes de carbone, les huiles fluorées partiellement hydrocarbonées et/ou siliconées, les huiles siliconées volatiles ou non, linéaires ou cycliques, liquides ou pâteux à température ambiante, les pigments, les nacres, les charges, les colorants hydrosolubles, les colorants liposolubles, les agents de filmification, les tensioactifs, les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents propulseurs, les céramides et leurs mélanges.

Cette formulation est aussi caractérisée en ce qu'elle se présente sous la forme d'une composition capillaire, préférentiellement pour le maintien de la coiffure ou la mise en forme des cheveux, très préférentiellement sous forme de shampooings, de gels, de lotions de mise en plis, de lotions pour le brushing, de compositions de fixation et de coiffage choisies parmi les laques ou spray, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Un dernier objet de la présente invention consiste un procédé cosmétique de traitement des cheveux, caractérisé en ce qu'il consiste à appliquer sur les cheveux une formulation cosmétique telle que définie précédemment.

Les exemples qui suivent permettront de mieux appréhender l'invention, sans pour autant en limiter la portée.

### EXEMPLES

On prépare une composition de shampoing comprenant les constituants suivants (% en poids):
- 7,5 % de lauryl éther sulfate,
- 2,5 % de tensioactif amphotère coco-bétaïne (Dehyton AB30 de Cognis™),
- 5 % de tensioactif coco-polyglucoside (Plantacare 818 UP de Cognis™),
- 3% (en poids sec) du polymère à tester,
- qsp 100 % eau.

### Essai n° 1

Cet essai illustre l'art antérieur, et met en oeuvre un copolymère constitué de, exprimé en % en poids de chacun de ses monomères :
a) 20 % d'acide méthacrylique,
b) 80 % d'un macromonomère de formule (I) :

   R-(OP)ₘ-(OE)ₙ-R' (I)

   - m = 0, n = 48,
   - OP et OE désignent respectivement l'oxyde de propylène et l'oxyde d'éthylène,
   - R désigne la fonction méthacrylate,
   - R' représente le radical méthyle.
totalement neutralisé par la soude et de masse moléculaire moyenne en poids égale à 25 000 g/mol.

Le macromonomère (I) est donc ici le méthacrylate de MPEG de masse molaire moyenne en poids égale à 2 000 g/mol (illustré dans les documents EP 1 632 508 A1 et EP 2 168 991 A1).

### Essai n° 2

Cet essai illustre l'art antérieur, et met en oeuvre un copolymère constitué de, exprimé en % en poids de chacun de ses monomères :
a) 20 % d'acide méthacrylique,
b) 80 % d'un macromonomère de formule (I) :

   R-(OP)ₘ-(OE)ₙ-R' (I)

   - m = 0, n = 110,
   - OP et OE désignent respectivement l'oxyde de propylène et l'oxyde d'éthylène,
   - R désigne la fonction méthacrylate,
   - R' représente le radical méthyle,
totalement neutralisé par la soude et de masse moléculaire moyenne en poids égale à 60 000 g/mol.

Le macromonomère (I) est donc ici le méthacrylate de MPEG de masse molaire moyenne en poids égale à 5 000 g/mol.

### Essai n° 3

Cet essai illustre l'invention, et met en oeuvre un copolymère constitué de, exprimé en % en poids de chacun de ses monomères :
a) 20 % d'acide méthacrylique,
b) 80 % d'un macromonomère de formule (I):

   R-(OP)ₘ-(OE)ₙ-R' (I)

   - m = 15, n = 46,
   - OP et OE désignent respectivement l'oxyde de propylène et l'oxyde d'éthylène,
   - R désigne la fonction méthacrylate,
   - R' représente l'hydrogène,
totalement neutralisé par la soude et de masse moléculaire moyenne en poids égale à 60 000 g/mol.

On constate en milieux humide et sec de bonnes propriétés de démêlage, et un coiffage amélioré dans le cas du polymère n° 3 selon l'invention. On constate aussi une aptitude plus prononcée pour ce polymère à être éliminé par rinçage des cheveux sous l'eau.

### Essai n° 4

Cet essai illustre l'invention, et met en oeuvre un copolymère constitué de, exprimé en % en poids de chacun de ses monomères :
a) 8 % d'acide acrylique, 2,5 % d'acide méthacrylique,
b) 89,5 % d'un macromonomère de formule (I) :

   R-(OP)ₘ-(OE)ₙ-R' (I)

   - m = 15, n = 46,
   - OP et OE désignent respectivement l'oxyde de propylène et l'oxyde d'éthylène,
   - R désigne la fonction méthacrylate,
   - R' représente l'hydrogène,
totalement neutralisé par la soude et de masse moléculaire moyenne en poids égale à 120 000 g/mol.

### Essai n° 5

Cet essai illustre l'art antérieur, et met en oeuvre un copolymère constitué de, exprimé en % en poids de chacun de ses monomères :
a) 6 % d'acide acrylique, 1,8 % d'acide méthacrylique,
b) 92,2 % d'un macromonomère de formule (I) :

   R-(OP)ₘ-(OE)ₙ-R' (I)

   - m = 0, n = 45,
   - OP et OE désignent respectivement l'oxyde de propylène et l'oxyde d'éthylène,
   - R désigne la fonction méthacrylate,
   - R' représente le radical méthyle,
totalement neutralisé par la soude et de masse moléculaire moyenne en poids égale à 100 000 g/mol.

Les polymères selon les essais n° 4 et 5 ont également été testés dans la formulation de shampoing décrite en introduction des exemples.

On constate en milieux humide et sec de bonnes propriétés de démêlage, et un coiffage amélioré dans le cas du polymère n° 4 selon l'invention, à un niveau comparable à celui obtenu avec le polymère selon l'essai n° 3. On constate aussi une aptitude plus prononcée pour ce polymère à être éliminé par rinçage des cheveux sous l'eau.
En comparaison, les propriétés obtenues avec le polymère selon l'essai n° 5 restent comparables à celles obtenues avec les polymères selon les essais n° 1 et 2.

## Revendications

1. Utilisation comme agent coiffant, dans une formulation cosmétique, d'au moins un copolymère (méth)acrylique peigne constitué de :
a) de 5 % à 30 % d'au moins un monomère qui est l'acide (méth)acrylique, et
b) de 70 % à 95 % d'au moins un macromonomère de formule (I) :
R-(OP)ₘ-(OE)ₙ-R' (I)
dans laquelle :
- m et n sont des entiers non nuls inférieurs à 150, n et m étant compris entre 10 et 90,
- OP et OE désignent respectivement l'oxyde de propylène et l'oxyde d'éthylène,
- R désigne une fonction insaturée polymérisable,
- R' représente l'hydrogène ou un groupement alkyle ayant de 1 à 4 atomes de carbone.

2. Utilisation selon la revendication 1, selon laquelle le copolymère (méth)acrylique peigne est constitué, exprimé en pourcentage en poids de chacun de ses constituants :
a) de 15 % à 25 % d'au moins un monomère qui est l'acide (méth)acrylique,
b) de 75 % à 85 % d'au moins un macromonomère de formule (I),
c) de 0 % à 20 %, préférentiellement de 0 % à 10 % d'au moins un monomère qui est un ester de l'acide (méth)acrylique, préférentiellement l'acrylate d'éthyle,la somme des pourcentages a), b) et c) étant égale à 100 %.

3. Utilisation selon une des revendications 1 ou 2, selon laquelle R désigne la fonction méthacrylate ou méthacryluréthane.

4. Utilisation selon une des revendications 1 à 3, selon laquelle R' désigne l'hydrogène.

5. Utilisation selon une des revendications 1 à 4, selon laquelle le copolymère (méth)acrylique peigne présente une masse molaire moyenne en poids comprise entre 20 000 g/mol et 6 000 000 g/mol, préférentiellement entre 40 000 g/mol et 1 000 000 g/mol.

6. Formulation cosmétique contenant au moins un copolymère (méth)acrylique peigne constitué de :
a) de 5 % à 30 % d'au moins un monomère qui est l'acide (méth)acrylique, et
b) de 70 % à 95 % d'au moins un macromonomère de formule (I) :
R-(OP)ₘ-(OE)ₙ-R' (I)
dans laquelle :
- m et n sont des entiers non nuls inférieurs à 150, n et m étant compris entre 10 et 90,
- OP et OE désignent respectivement l'oxyde de propylène et l'oxyde d'éthylène,
- R désigne une fonction insaturée polymérisable,
- R' représente l'hydrogène ou un groupement alkyle ayant de 1 à 4 atomes de carbone.

7. Formulation selon la revendication 6, dans laquelle le copolymère (méth)acrylique peigne est constitué, exprimé en pourcentage en poids de chacun de ses constituants :
a) de 15 % à 25 % d'au moins un monomère qui est l'acide (méth)acrylique,
b) de 75 % à 85 % d'au moins un macromonomère de formule (I),
c) de 0 % à 20 %, préférentiellement de 0 % à 10 % d'au moins un monomère qui est un ester de l'acide (méth)acrylique, préférentiellement l'acrylate d'éthyle,
la somme des pourcentages a), b) et c) étant égale à 100 %.

8. Formulation selon une des revendications 6 ou 7, dans laquelle, pour ledit copolymère, R désigne la fonction méthacrylate ou méthacryluréthane.

9. Formulation selon une des revendications 6 à 8, dans laquelle, pour ledit copolymère, R' désigne l'hydrogène.

10. Formulation selon une des revendications 6 à 9, dans laquelle ledit copolymère présente une masse molaire moyenne en poids comprise entre 20 000 g/mol et 6 000 000 g/mol, préférentiellement entre 40 000 g/mol et 1 000 000 g/mol.

11. Formulation selon une des revendications 6 à 10, comprenant de 0,01 % à 50 %, préférentiellement de 0,1 % à 30 % très préférentiellement de 3 % à 20% en poids sec dudit copolymère par rapport à son poids total.

12. Formulation selon une des revendications 6 à 11, comprenant au moins un constituant choisi parmi l'eau, les solvants organiques hydrophiles et préférentiellement les alcools, préférentiellement les monoalcools, linéaires ou ramifiés en C1-C6 et les polyols et les éthers de glycols préférentiellement en C2 et des aldéhydes en C2-C4 hydrophiles, les cires, les corps gras pâteux, les gommes et leurs mélanges, d'origine animale, végétale, minérale ou synthétique, les solvants organiques lipophiles, les huiles d'origine animale, végétale, minérale ou synthétique, les esters et les éthers de synthèse, les esters du pentaérythritol, les alcools gras ayant de 12 à 26 atomes de carbone, les huiles fluorées partiellement hydrocarbonées et/ou siliconées, les huiles siliconées volatiles ou non, linéaires ou cycliques, liquides ou pâteux à température ambiante, les pigments, les nacres, les charges, les colorants hydrosolubles, les colorants liposolubles, les agents de filmification, les tensioactifs, les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents propulseurs, les céramides et leurs mélanges.

13. Formulation selon une des revendications 6 à 12, **caractérisée en ce qu'**elle se présente sous la forme d'une composition capillaire, préférentiellement pour le maintien de la coiffure ou la mise en forme des cheveux, très préférentiellement sous forme de shampooings, de gels, de lotions de mise en plis, de lotions pour le brushing, de compositions de fixation et de coiffage choisies parmi les laques ou spray, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

14. Procédé cosmétique de traitement des cheveux, consistant à appliquer sur les cheveux une formulation cosmétique selon une des revendications 6-13.

## Patentansprüche

1. Verwendung als Frisiermittel in einer Kosmetikformulierung mindestens eines (Meth)Acryl-Kamm-Copolymers, das aus Folgendem besteht:
a) 5% bis 30% mindestens eines Monomers, das (Meth)Acrylsäure ist, und
b) 70% bis 95% mindestens eines Makromonomers der Formel (I):
R-(OP)ₘ-(OE)ₙ-R' (I)
worin:
- m und n von null verschiedene ganze Zahlen kleiner als 150 sind, wobei n und m zwischen 10 und 90 liegen,
- OP und OE Propylenoxid bzw. Ethylenoxid bezeichnen,
- R eine ungesättigte polymerisierbare Funktion bezeichnet,
- R' für Wasserstoff oder eine Alkylgruppe mit 1 bis 4 Kohlenstoffatomen steht.

2. Verwendung nach Anspruch 1, wobei das (Meth)Acryl-Kamm-Copolymer, ausgedrückt in Gewichtsprozent jedes seiner Bestandteile, aus Folgendem besteht:
a) 15% bis 25% mindestens eines Monomers, das (Meth)Acrylsäure ist,
b) 75% bis 85% mindestens eines Makromonomers der Formel (I),
c) 0% bis 20%, vorzugsweise 0% bis 10% mindestens eines Monomers, das ein (Meth)Acrylsäureester, vorzugsweise Ethylacrylat ist,
wobei die Summe der Prozente von a), b) und c) gleich 100% ist.

3. Verwendung nach einem der Ansprüche 1 oder 2, wobei R für die Methacrylat- oder Methacrylurethanfunktion steht.

4. Verwendung nach einem der Ansprüche 1 bis 3, wobei R' für Wasserstoff steht.

5. Verwendung nach einem der Ansprüche 1 bis 4, wobei das (Meth)Acryl-Kamm-Copolymer eine gewichtsgemittelte Molmasse zwischen 20 000 g/mol und 6 000 000 g/mol, vorzugsweise zwischen 40 000 g/mol und 1 000 000 g/mol aufweist.

6. Kosmetikformulierung, die mindestens ein (Meth)Acryl-Kamm-Copolymer enthält, das aus Folgendem besteht:
a) 5% bis 30% mindestens eines Monomers, das (Meth)Acrylsäure ist, und
b) 70% bis 95% mindestens eines Makromonomers der Formel (I):
R-(OP)ₘ-(OE)ₙ-R' (I)
worin:
- m und n von null verschiedene ganze Zahlen kleiner als 150 sind, wobei n und m zwischen 10 und 90 liegen,
- OP und OE Propylenoxid bzw. Ethylenoxid bezeichnen,
- R eine ungesättigte polymerisierbare Funktion bezeichnet,
- R' für Wasserstoff oder eine Alkylgruppe mit 1 bis 4 Kohlenstoffatomen steht.

7. Formulierung nach Anspruch 6, wobei das (Meth)Acryl-Kamm-Copolymer, ausgedrückt in Gewichtsprozent jedes seiner Bestandteile, aus Folgendem besteht:
a) 15% bis 25% mindestens eines Monomers, das (Meth)Acrylsäure ist,
b) 75% bis 85% mindestens eines Makromonomers der Formel (I),
c) 0% bis 20%, vorzugsweise 0% bis 10% mindestens eines Monomers, das ein (Meth)Acrylsäureester, vorzugsweise Ethylacrylat ist,
wobei die Summe der Prozente von a), b) und c) gleich 100% ist.

8. Formulierung nach einem der Ansprüche 6 oder 7, wobei für das Copolymer R die Methacrylat- oder Methacrylurethanfunktion bezeichnet.

9. Formulierung nach einem der Ansprüche 6 bis 8, wobei für das Copolymer R' Wasserstoff bezeichnet.

10. Formulierung nach einem der Ansprüche 6 bis 9, wobei das Copolymer eine gewichtsgemittelte Molmasse zwischen 20 000 g/mol und 6 000 000 g/mol, vorzugsweise zwischen 40 000 g/mol und 1 000 000 g/mol aufweist.

11. Formulierung nach einem der Ansprüche 6 bis 10, die 0,01% bis 50%, vorzugsweise 0,1% bis 30%, ganz besonders bevorzugt 3% bis 20% Trockenmasse des Copolymers, bezogen auf ihr Gesamtgewicht, umfasst.

12. Formulierung nach einem der Ansprüche 6 bis 11, die mindestens einen Bestandteil umfasst, ausgewählt aus Wasser, hydrophilen organischen Lösungsmitteln und vorzugsweise Alkoholen, bevorzugt geraden oder verzweigten C1-C6-Monoalkoholen und Polyolen und, bevorzugt C2-, Glykolethern und hydrophilen C2-C4-Aldehyden, Wachsen, pastösen Fetten, Gummen und ihren Gemischen tierischen, pflanzlichen, anorganischen oder synthetischen Ursprungs, lipophilen organischen Lösungsmitteln, Ölen tierischen, pflanzlichen, anorganischen oder synthetischen Ursprungs, Syntheseestern und -ethern, Pentaerythritestern, Fettalkoholen mit 12 bis 26 Kohlenstoffatomen, fluorierten, partiellen Kohlenwasserstoff- und/oder Silikonölen, flüchtigen oder nichtflüchtigen, geraden oder zyklischen, bei Umgebungstemperatur flüssigen oder pastösen Silikonölen, Pigmenten, Perlmuttern, Füllstoffen, wasserlöslichen Farbstoffen, fettlöslichen Farbstoffen, filmbildenden Mitteln, Tensiden, Vitaminen, Parfums, Perlglanzmitteln, Verdickungsmitteln, Geliermitteln, Spurenelementen, Weichmachern, Sequestrierungsmitteln, Parfums, Alkalinisierungs- oder Ansäuerungsmitteln, Konservierungsmitteln, Sonnenschutzmitteln, Antioxidantien, Mitteln gegen Haarausfall, Antischuppenmitteln, Treibmitteln, Ceramiden und deren Gemischen.

13. Formulierung nach einem der Ansprüche 6 bis 12, **dadurch gekennzeichnet, dass** sie in Form einer Haarzusammensetzung vorliegt, vorzugsweise zur Erhaltung der Frisur oder zum Formen der Haare, ganz besonders bevorzugt in Form von Shampoos, Gelen, Lockenformungslotionen, Föhnstylinglotionen, Festiger- und Frisierzusammensetzungen, ausgewählt aus Lacken oder Spray, Spülung zum Ausspülen oder Nicht-Ausspülen, Dauerwellen-, Glättungs-, Färbungs- oder Entfärbungszusammensetzungen, oder auch in Form von Spülzusammensetzungen für die Anwendung vor oder nach einer Färbung, einer Entfärbung, einer Dauerwelle oder Glättung oder auch zwischen den beiden Schritten einer Dauerwelle oder Glättung.

14. Kosmetisches Haarbehandlungsverfahren, das aus dem Aufbringen einer Kosmetikformulierung nach einem der Ansprüche 6 bis 13 auf die Haare besteht.

## Claims

1. A use as a hair styling agent in a cosmetic formulation of at least one (meth)acrylic comb copolymer made up of:
a) from 5% to 30% of at least one monomer which is (meth)acrylic acid, and
b) from 70% to 95% of at least one macromonomer of formula (I):
R-(PO)ₘ-(EO)ₙ-R' (I)
in which:
- m and n are non-zero integers less than 150, n and m being between 10 and 90,
- PO and EO respectively designate propylene oxide and ethylene oxide,
- R designates a polymerizable unsaturated group,
- R' represents hydrogen or an alkyl group with from 1 to 4 carbon atoms.

2. The use according to claim 1, according to which the (meth)acrylic comb copolymer is made up of, expressed as a percentage by weight of each of its constituents:
a) from 15% to 25% of at least one monomer which is (meth)acrylic acid,
b) from 75% to 85% of at least one macromonomer of formula (I),
c) from 0% to 20%, preferentially form 0% to 10% of at least one monomer which is an ester of (meth)acrylic acid, preferentially ethyl acrylate,
the sum of the percentages a), b) and c) being equal to 100%.

3. The use according to one of claims 1 or 2, according to which R designates the methacrylate or methacrylurethane function.

4. The use according to one of claims 1 to 3, according to which R' designates hydrogen.

5. The use according to one of claims 1 to 4, according to which the (meth)acrylic comb copolymer has a mean molar mass by weight of between 20,000 g/mol and 6,000,000 g/mol, preferentially between 40,000 g/mol and 1,000,000 g/mol.

6. A cosmetic formulation containing at least one (meth)acrylic comb copolymer made up of:
c) from 5% to 30% of at least one monomer which is (meth)acrylic acid,
d) from 70% to 95% of at least one macromonomer of formula (I):
R-(PO)ₘ-(EO)ₙ-R' (I)
in which:
- m and n are non-zero integers less than 150, n and m being comprised between 10 and 90,
- PO and EO respectively designate propylene oxide and ethylene oxide,
- R designates a polymerizable unsaturated function,
- R' represents hydrogen or an alkyl group with from 1 to 4 carbon atoms.

7. The formulation according to claim 6, in which the (meth)acrylic comb copolymer is made up of, expressed as a percentage by weight of each of its constituents:
a) from 15% to 25% of at least one monomer which is (meth)acrylic acid,
b) from 75% to 85% of at least one macromonomer of formula (I),
c) from 0% to 20%, preferentially form 0% to 10% of at least one monomer which is an ester of (meth)acrylic acid, preferentially ethyl acrylate,
the sum of the percentages a), b) and c) being equal to 100%.

8. The formulation according to one of claims 6 or 7, in which, for said copolymer, R designates the methacrylate or methacrylurethane function.

9. The formulation according to one of claims 6 to 8, in which, for said copolymer, R' designates hydrogen.

10. The formulation according to one of claims 6 to 9, in which said copolymer has a mean molar mass by weight of between 20,000 g/mol and 6,000,000 g/mol, preferentially between 40,000 g/mol and 1,000,000 g/mol.

11. The formulation according to one of claims 6 to 10, comprising from 0.01% to 50%, preferentially from 0.1% to 30%, very preferentially from 3% to 20% by dry weight of said copolymer based on its total weight.

12. The formulation according to one of claims 6 to 11, comprising at least one constituent chosen from among water, hydrophilic organic solvents and preferentially alcohols, preferentially linear or branched C1-C6 monoalcohols, and polyols and glycol ethers, preferentially in C2, and hydrophilic aldehydes in C2-C4, waxes, pasty fats, gums and their mixtures, of animal, vegetable, mineral or synthetic origin, lipophilic organic solvents, oils of animal, vegetable, mineral or synthetic origin, synthesis esters and ethers, pentaerythritol esters, the fatty alcohols with 12 to 26 carbon atoms, fluorinated oils partially hydrocarbonated and/or siliconated, volatile or not volatile, linear or cyclic, liquid or pasty at room temperature siliconated oils, pigments, nacres, fillers, water-soluble dyes, fat-soluble dyes, filmification agents, surfactants, vitamins, perfumes, nacrating agents, thickeners, gelling agents, trace elements, softeners, sequestrating aids, perfumes, alkalinizing or acidifying agents, preservatives, solar filters, anti-oxidants, the anti hair loss agents, anti-dandruff agents, propellant agents, ceramides and their mixtures.

13. The formulation according to one of claims 6 to 12, **characterized in that** it is in the form of a capillary composition, preferentially for the maintenance of hair styling or the shaping of hair, very preferentially in the form of shampoos, gels, waving lotions, brushing lotions, of setting and styling compositions chosen from the lacquers or sprays, after-shampoos whether rinsed or not, compositions for permanents, straightening, dyeing or bleaching, or again in the form of compositions for rinsing, for application before or after a dyeing, a bleaching, a permanent or a straightening, or again between the two steps of a permanent or a straightening.

14. A cosmetic hair treatment method, consisting of the application to the hair of a cosmetic formulation according to one of claims 6 to 13.
